# EUROPEAN PATENT APPLICATION

(11) **EP 4 582 068 A1**
(43) Date of publication of application: **09.07.2025**
(21) Application number: 25150497.3
(22) Date of filing: 07.01.2025
(51) Int. Cl.: A61F 7/02, A61F 7/03, A61K 8/02, A61K 8/18, A61F 7/00

(54) **SELF-RETRACTABLE PATCH FOR SKIN STIMULATION**

(30) Priority: 08.01.2024 KR 20240002801
(71) Applicant: Amorepacific Corporation, Seoul 04386 (KR)
(72) Inventor: Han, Kyungsup, 17074 Yongin-si (KR); Lee, Jeongyu, 17074 Yongin-si (KR); Lee, Jeongin, 17074 Yongin-si (KR)
(74) Representative: Schön, Christoph

(57) **Abstract**

The present disclosure relates to a self-retractable patch for skin stimulation, and more specifically, to a patch for skin stimulation including a retractable material and a trigger material. According to one embodiment of the present disclosure, as a patch capable of stimulating skin, a patch for skin stimulation including a retractable material capable of retracting under a preset condition and a trigger material which applies a preset energy to the retractable material so as to achieve the condition may be provided.

## Description

### CROSS-REFERENCE TO RELATED APPLICATION(S)

This application is based on and claims priority of Korean Patent Application No. 10-2024-0002801, filed on January 8, 2024 with the Korean Intellectual Property Office, the entire contents of which are hereby incorporated by reference.

### TECHNICAL FIELD

The present disclosure relates to a self-retractable patch for skin stimulation, and more specifically, to a patch for skin stimulation including a retractable material and a trigger material.

### BACKGROUND

Recently, as interest in beauty has increased rapidly, cosmetics having a form that may physically stimulate skin are being developed. In particular, cosmetics in form of a patch that stimulates the skin using physical retraction force to provide skin improvement effects are attracting attention.

Conventionally, in order to cause physical retraction, it was required to have an external device capable of causing the retraction or an energy source capable of supplying an energy required for the retraction. However, in the case of the conventional method, there is a problem that both customer's accessibility to purchase products and convenience of use are reduced because the external device or the energy source should be prepared.

In order to solve these problems, a method of memorizing or recording elongation in a patch in advance may be considered so that retraction may occur at a desired time without an additional external device or energy source. However, in this case, a problem that it is difficult to control a direction and degree of retraction may occur because the retraction of the patch is performed only in a direction in which the elongation is recorded. In addition, since an elongation process is included in a manufacturing process, the manufacturing process is very complicated and the manufacturing cost may be greatly increased.

### SUMMARY

A self-retractable patch for skin stimulation according to one embodiment of the present disclosure has been devised to solve the aforementioned problems, and is directed to providing of a patch for skin stimulation in which a direction and degree of retraction may be controlled.

In addition, the present disclosure is directed to providing of a patch for skin stimulation which allows a user to retract the patch at a desired time.

In addition, the present disclosure is directed to providing of a patch for skin stimulation with a simple manufacturing process and low manufacturing costs.

According to one embodiment of the present disclosure, as a patch capable of stimulating skin, a patch for skin stimulation including a retractable material capable of retracting under a preset condition and a trigger material which applies a preset energy to the retractable material so as to achieve the condition may be provided.

In addition, the patch for skin stimulation in which the preset energy is a thermal energy may be provided.

In addition, the patch for skin stimulation in which the patch is a hydrogel patch may be provided.

In addition, the patch for skin stimulation in which the retractable material is Poly(N-isopropylacrylamide) (PNIPAAm) may be provided.

In addition, the patch for skin stimulation in which the trigger material is a water-sensitive self-heating material may be provided.

In addition, the patch for skin stimulation in which the trigger material includes zeolite may be provided.

In addition, the patch for skin stimulation in which the trigger material further includes polyol may be provided.

In addition, the patch for skin stimulation including one or more holes formed in a region that is in contact with the skin may be provided.

In addition, the patch for skin stimulation may be provided in which the hole is formed in a circular shape having a diameter of 1 to 5 times an average skin pore size, a plurality of holes are provided, and an interval between any two holes adjacent to each other among the plurality of holes is formed to be 3 to 5 times the diameter of the hole.

According to one embodiment of the present disclosure, a patch for skin stimulation may include a retractable material capable of retracting under a preset condition and a warming patch including a trigger material which supplies a thermal energy to the retractable patch so as to achieve the condition, and the retractable patch and the warming patch may be separately provided to a user before use and used in combination with each other in use.

In addition, the patch for skin stimulation in which the retractable material is PNIPAAm may be provided.

In addition, the patch for skin stimulation in which the trigger material is a water-sensitive self-heating material may be provided.

In addition, the patch for skin stimulation in which the trigger material includes zeolite may be provided.

In addition, the patch for skin stimulation in which the trigger material further includes polyol may be provided.

According to one embodiment of the present disclosure, a patch for skin stimulation including a first layer including the retractable material capable of retracting under a preset condition and a second layer provided on one side or both sides of the first layer and including a trigger material which applies a preset energy so as to achieve the condition may be provided.

In addition, the patch for skin stimulation in which the preset energy is a thermal energy may be provided.

In addition, the patch for skin stimulation in which the retractable material is PNIPAAm may be provided.

In addition, the patch for skin stimulation in which the trigger material is a water-sensitive self-heating material may be provided.

In addition, the patch for skin stimulation in which the trigger material includes zeolite may be provided.

In addition, the patch for skin stimulation in which the trigger material further includes polyol may be provided.

According to a self-retractable patch for skin stimulation according to one embodiment of the present disclosure, there is an effect that it is possible to control a direction and degree of retraction.

In addition, there is an advantage that it is possible to retract the patch at a user's desired time.

In addition, there is an effect that a manufacturing process is simple, and a unit manufacturing cost can be lowered.

### BRIEF DESCRIPTION OF DRAWINGS

FIG. 1A is a plan view of a patch for skin stimulation according to a first embodiment of the present disclosure.
FIG. 1B is a plan view showing a modified example of the patch for skin stimulation of FIG. 1A.
FIG. 2 is a side cross-sectional view of a part of the patch for skin stimulation according to the first embodiment of the present disclosure.
FIG. 3 is a graph and table showing an exothermic reaction duration of a trigger material according to the first embodiment of the present disclosure.
FIG. 4 is an experimental example showing a degree of retraction according to time change of the patch for skin stimulation according to the first embodiment of the present disclosure.
FIG. 5A is a plan view showing the appearance of a conventional patch retracting.
FIG. 5B is a plan view showing a state in which the patch according to the first embodiment of the present disclosure retracts.
FIG. 6A is, as a side cross-sectional view of a part of a patch for skin stimulation according to a second embodiment of the present disclosure, a view showing a retractable patch and a warming patch are provided separately before use.
FIG. 6B is a view showing a state in which the retractable patch and the warming patch of FIG. 6A are used in combination with each other in use.
FIG. 7 is a side cross-sectional view of a part of a patch for skin stimulation according to a third embodiment of the present disclosure.
FIG. 8 is a side cross-sectional view of a part of a patch for skin stimulation according to a modified example of the third embodiment of the present disclosure.

### DETAILED DESCRIPTION OF EXEMPLARY EMBODIMENTS

The present disclosure may be modified in various ways and have many embodiments, and specific embodiments are exemplified and described in detail in the detailed description. However, this is not intended to limit the present disclosure to specific embodiments, but should be understood to include all transformations, equivalents, or substitutes included in the spirit and technical scope of the present disclosure.

The terminology used in the present disclosure is only used to describe specific embodiments and is not intended to limit the present disclosure. Singular expressions include plural expressions unless the context clearly indicates otherwise. In the present disclosure, it should be understood that terms such as "include" or "have" are intended to specify the presence of a feature, number, step, operation, component, part or combination thereof described in the specification, but do not exclude in advance the possibility of the presence or addition of one or more other features, numbers, steps, operations, components, parts or combinations thereof.

Hereinafter, preferred embodiments of the present disclosure will be described in detail with reference to the accompanying drawings. In this case, it should be noted that in the accompanying drawings, identical components are represented by identical symbols where possible. In addition, detailed descriptions of known functions and configurations that may obscure the gist of the present disclosure will be omitted. For the same reason, some components in the accompanying drawings are exaggerated, omitted, or schematically depicted.

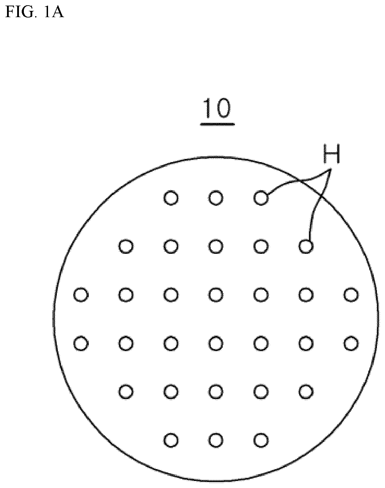

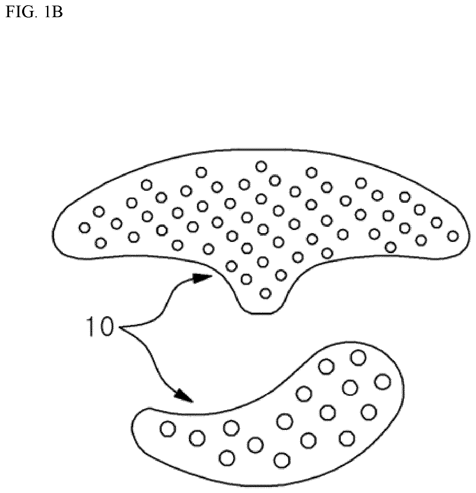

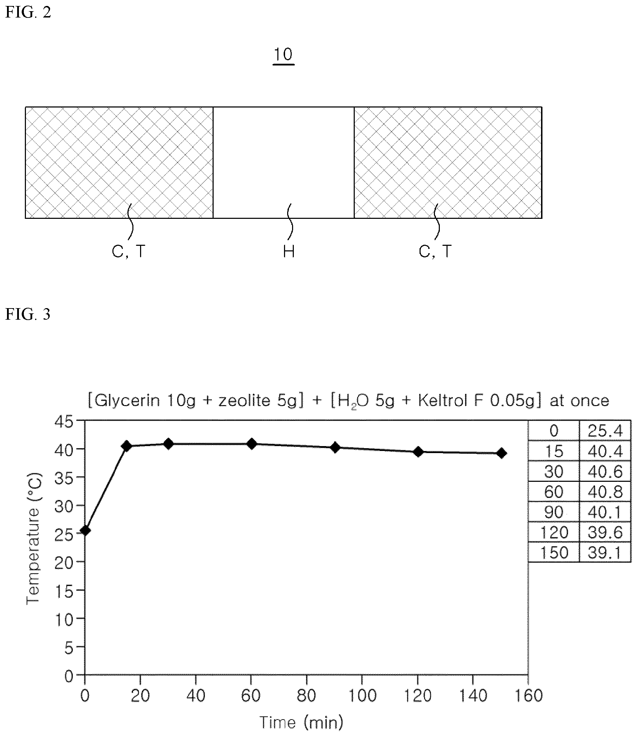

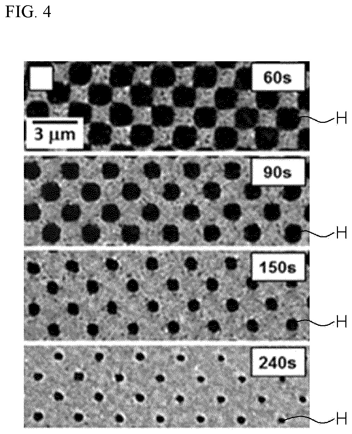

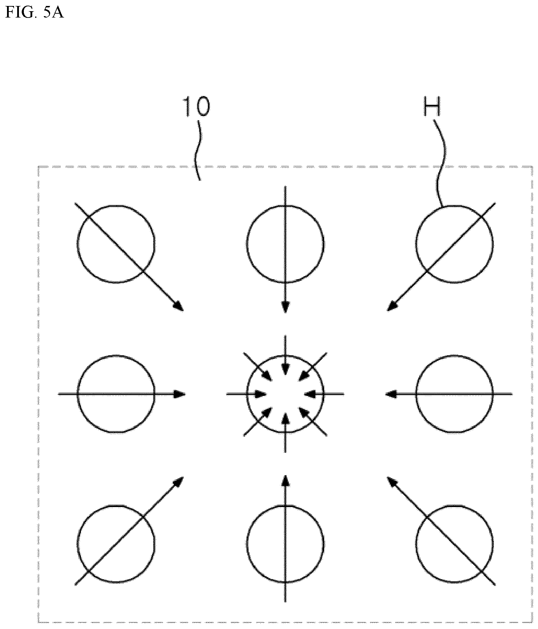

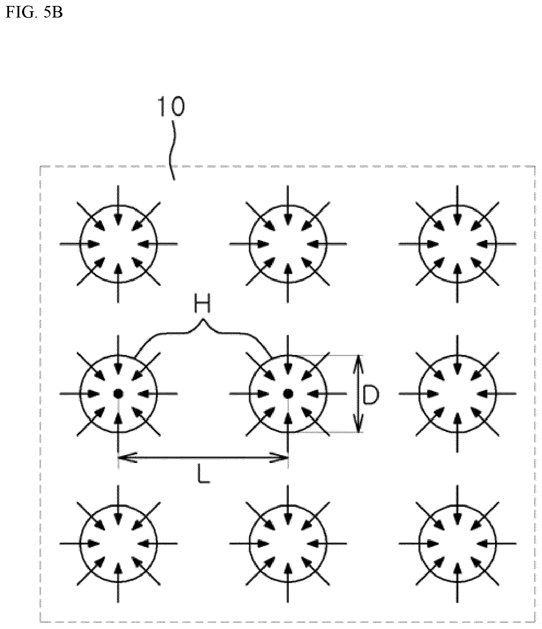

FIG. 1A is a plan view of a patch for skin stimulation according to a first embodiment of the present disclosure, FIG. 1B is a plan view showing a modified example of the patch for skin stimulation of FIG. 1A, FIG. 2 is a side cross-sectional view of a part of the patch for skin stimulation according to the first embodiment of the present disclosure, FIG. 3 is a graph and table showing an exothermic reaction duration of a trigger material according to the first embodiment of the present disclosure, FIG. 4 is an experimental example showing a degree of retraction according to time change of the patch for skin stimulation according to the first embodiment of the present disclosure, FIG. 5A is a plan view showing the appearance of a conventional patch retracting, and FIG. 5B is a plan view showing a state in which the patch according to the first embodiment of the present disclosure retracts.

Hereinafter, a patch for skin stimulation 10 according to the first embodiment of the present disclosure will be described in detail with reference to FIGS. 1A to 5B. The patch for skin stimulation 10 (hereinafter, used interchangeably with a "patch 10") according to the first embodiment of the present disclosure may include a retractable material C and a trigger material T. The patch 10 according to the first embodiment of the present disclosure may be provided in a state in which the retractable material C and the trigger material T are mixed.

The patch 10 according to the first embodiment of the present disclosure may be attached to the skin and may be retracted while attached to the skin. The patch 10 may be provided in a circular shape as a whole as shown in FIG. 1A. In addition, the patch 10 may be provided in various shapes, such as a non-circular shape as shown in FIG. 1B, and in this case, the patch 10 may be easily attached to a specific part of a user's face.

The retractable material C is a material that may be retracted under a preset condition. Here, it may be understood that the preset condition is a condition in which a specific energy is applied to the retractable material C and thus the retractable material C is placed in a specific physical, electrical, or chemical state. In the case of the first embodiment of the present disclosure, the condition may be understood as a condition in which the retractable material C is placed in a state of specific temperature. For example, when the retractable material C becomes a state having a temperature of 37 degrees Celsius or more, the retractable material C may be retracted.

The trigger material T may be a material that enables the condition to be achieved and apply a preset energy to the retractable material C. The trigger material T may apply a physical, electrical, or chemical energy to the retractable material C. In the case of the first embodiment of the present disclosure, the preset energy may be a thermal energy. For example, the trigger material T may supply thermal energy to the retractable material C so that the retractable material C is in a state of a specific temperature.

In one embodiment, the retractable material C may be Poly(N-isopropylacrylamide) (PNIPAAm). The PNIPASm is a material which is a type of polymer compound, has both hydrophilic and hydrophobic groups, and exhibits a characteristic of volume change or temperature-sensitive swelling behavior at a temperature similar to the body temperature. The PNIPAAm may undergo retraction, for example, when placed at a temperature of 37 degrees Celsius or more.

In one embodiment, the trigger material T may be a self-heating material. The trigger material T may include a material that dissipates heat by itself. The heat dissipated from the trigger material T may be supplied to the retractable material C. When the retractable material C may include the PNIPAAm, the retractable material C may be placed at a temperature of 37 degrees Celsius or more and retracted by the heat supplied from the trigger material T.

In one embodiment, the trigger material T may be a water-sensitive self-heating material. Specifically, when supplied with water, the trigger material T may be a material that reacts with the water and dissipates heat by itself.

In one embodiment, the trigger material T is a self-heating material and may include a metal-based material. For example, the trigger material T may include at least any one of an oxygen-activated material which dissipates heat through an oxygen activation reaction such as a metal oxide, a metal powder such as aluminum (Al) powder, metal nanoparticles such as aluminum nanoparticles, a metal alloy, and zeolite.

In one embodiment, the trigger material T is a self-heating material and may include zeolite. The zeolite may be understood as a general term for minerals in which alkali metals and alkali earth metals are combined with anions generated by the combination of aluminum oxide and silicate oxide. The zeolite may adsorb moisture. When the zeolite adsorbs moisture, the zeolite may cause an exothermic reaction to dissipate heat. That is, when the trigger material T includes zeolite, it may absorb moisture and dissipate heat to the outside through an exothermic reaction in the process.

In one embodiment, the trigger material T may include polyol. The polyol may increase the exothermic duration of the trigger material T. The higher the content of the polyol, the more the exothermic duration of the trigger material T may be increased, and the trigger material T may optimally design the exothermic duration of the trigger material T by further including thickener.

The polyol is at least one selected from a group consisting of butylene glycol, glycerin, propanediol, pentylene glycol, 1,2-hexanediol, and ethyl hexanediol, and may preferably be butylene glycol or propanediol, but is not limited thereto.

Meanwhile, the trigger material T may include a material that dissipates heat through interaction with the skin. For example, the trigger material T may include capsaicin. Capsaicin is a compound that causes a spicy taste, stimulates the epithelium of the skin when being in contact with the skin, and may dissipate heat through interaction with the skin.

In addition, the trigger material T may contain various chemical materials that generate heat through exothermic reaction under a specific condition. For example, the trigger material T may include a chemical material that generates heat by various exothermic reactions such as heat of dissolution generated during a melting process, heat of solidification or heat of liquefaction generated during a phase change process such as solidification or liquefaction, heat of neutralization through an acid-base reaction, and heat generated by an oxidation-reduction reaction of a metal.

FIG. 3 shows a change in temperature (in Celsius) of the trigger material T over time (min) when the trigger material T includes 5 g of zeolite and 10 g of glycerin, and at the same time, 5 g of water (H2O) and 0.05 g of a thickener (Keltrol F) are added to the trigger material T. Referring to the left graph and right table of FIG. 3, it may be confirmed that the trigger material T maintains a temperature of 40 degrees Celsius or more from 15 minutes to 90 minutes and a temperature of 39 degrees Celsius or more from 90 minutes to 150 minutes. Accordingly, it may be seen that when the trigger material T is composed of zeolite and glycerin and moisture is supplied thereto, the exothermic reaction continues for a long time so that the temperature may be maintained at 37 degrees Celsius or more.

Moisture may be supplied to the trigger material T in various ways. Moisture may be supplied to the trigger material T when the patch 10 is attached to a user's skin or immediately before attachment. For example, when the patch 10 is attached to the skin, moisture contained in the skin itself may be supplied to the trigger material T. Alternatively, when applying a formulation containing moisture such as toner or lotion to a specific area of the skin immediately before attaching the patch 10 to the skin and then attaching the patch 10 to the specific area, the moisture contained in the formulation of the specific area may be supplied to the trigger material T. Alternatively, a method may be used in which moisture or the formulation containing moisture is separately supplied in advance to the trigger material T immediately before the patch 10 is attached to the user's skin, and then the patch 10 is attached to the skin immediately or after a certain period of time.

The patch 10 according to the first embodiment of the present disclosure may be formed as a hydrogel patch 10, that is, the patch 10 made of a hydrogel material. Hydrogel refers to a material capable of containing a large amount of moisture because a hydrophilic polymer has a three-dimensional cross-linked structure through a physical bond such as a hydrogen bond and an ionic bond or a chemical covalent bond.

The patch 10 according to the first embodiment of the present disclosure may include one or more holes H. In the patch 10, the hole H is formed in a region that is in contact with the skin. The hole H may be formed by penetrating a thickness direction of the patch 10.

The hole H may be retracted together when the patch 10 is retracted. The patch 10 according to the first embodiment of the present disclosure may include a plurality of holes H, and each of the plurality of holes H may be retracted in all two-dimensional directions. FIG. 4 shows an experimental example in which each hole H is retracted as a heat supply time passes (60, 90, 150, and 240 seconds) when heat is supplied to the patch 10. Accordingly, it may be seen that the hole H is retracted as the heat supply time continues, and it may be seen that all of each hole H are retracted while maintaining a certain shape so that all holes H are retracted in all two-dimensional directions.

With reference to FIGS. 5A and 5B, the retraction of a hole H of a conventional patch 10 and the patch 10 according to the first embodiment of the present disclosure will be examined in more detail. In FIG. 5A, a case where the hole H is formed on the conventional patch 10 is illustrated, and in FIG. 5B, the patch 10 according to the first embodiment of the present disclosure is illustrated. Referring to FIG. 5A, after first recording an elongation, the conventional patch 10 is retracted in a way that it is restored to an initial state before the elongation is recorded. In the case of such conventional patch 10, the patch 10 is retracted along a direction in which the elongation is recorded, and accordingly, a plurality of holes H are retracted along the direction in which the elongation is recorded in the patch 10 as well so that each hole H may not be uniformly retracted in a certain shape.

On the other hand, referring to FIG. 5B, since the patch 10 according to the first embodiment of the present disclosure is retracted by itself regardless of whether the elongation is recorded, the patch 10 may be retracted in all two-dimensional directions, not just along the direction in which the elongation is recorded. Accordingly, each hole H of the plurality of holes H may be retracted in all two-dimensional directions. In this case, the plurality of holes H may be retracted toward their respective centers.

In one embodiment, the hole H may be retracted at a retraction rate of 40% to 90%. Here, the retraction rate may be understood as a value obtained by subtracting a later size from an initial size of the hole H and then dividing the obtained value with the initial size of the hole H. For example, when a diameter of the hole H is 1000 µm and the retraction rate is 90%, the hole H may be retracted until the diameter reaches 100 µm. In this case, when an average skin pore size of human skin is 250 µm, the hole H may be retracted to less than 50% of the average skin pore size. Meanwhile, as the hole H is retracted, it may apply stimulation to the skin, such as narrowing skin pores.

In one embodiment, the hole H may be provided in a circular shape. The shape of the hole H is not limited thereto and may be provided in other shapes than the circular shape. A diameter D of the hole H may be formed to be 1 to 6 times the average skin pore size of human skin, and preferably 1 to 4 times. For example, when the average skin pore size is 250 µm, the diameter D of the hole H may be provided in a size of 250 µm to 1500 µm, and preferably in a size of 250 µm to 1000 µm.

When there are a plurality of holes H, an interval between any two holes H adjacent to each other among the plurality of holes H may be formed to be 3 to 5 times the diameter D of the hole H, and preferably 4 times. For example, when the diameter of the hole H is 250 µm, the interval between any two holes H adjacent to each other may be formed to be 1000 µm.

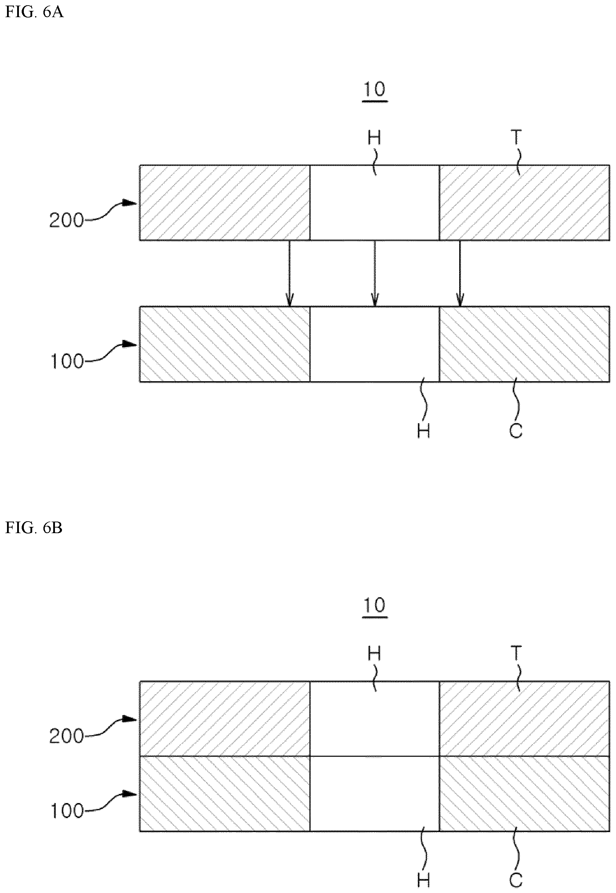

FIG. 6A is, as a side cross-sectional view of a part of a patch for skin stimulation according to a second embodiment of the present disclosure, a view showing a retractable patch and a warming patch are separately provided to a user before use, and FIG. 6B is a view showing a state in which the retractable patch and the warming patch of FIG. 6A are used in combination with each other in use.

Hereinafter, a patch for skin stimulation 10 according to the second embodiment of the present disclosure will be described in detail with reference to FIGS. 6A and 6B. The patch 10 according to the second embodiment of the present disclosure is different from the first embodiment of the present disclosure in that a retractable patch 100 and a warming patch 200 are separately provided to a user. Hereinafter, for convenience of description, duplicate descriptions with the first embodiment of the present disclosure will be omitted.

The patch for skin stimulation 10 according to the second embodiment of the present disclosure may include the retractable patch 100 and the warming patch 200.

The retractable patch 100 may include a retractable material C. The retractable material C is a material capable of retracting under a preset condition similar to the retractable material C of the patch 10 according to the first embodiment of the present disclosure, and as an example, the retractable material C may be Poly(N-isopropylacrylamide) (PNIPAAm).

The warming patch 200 may include a trigger material T. Similar to the trigger material T of the patch 10 according to the first embodiment of the present disclosure, the trigger material T may be a material that enables the condition to be achieved, may apply a specific energy to the retractable material C, and may be a water-sensitive self-heating material. The trigger material T may be a self-heating material and include zeolite. In addition, the trigger material T may include polyol.

The retractable patch 100 and the warming patch 200 may be separately provided to a user before use (FIG. 6A). That is, the retractable patch 100 and the warming patch 200 may be provided to be separated from each other before use. The retractable patch 100 and the warming patch 200 may be used in combination with each other in use (FIG. 6B). In the case where the retractable patch 100 and the warming patch 200 are combined, when heat is generated in the warming patch 200, the generated heat may be supplied from the warming patch 200 to the retractable patch 100.

The patch 10 according to the second embodiment of the present disclosure may include one or more holes H, similar to the patch 10 according to the first embodiment of the present disclosure. In this case, the hole H is formed in a region that is in contact with the skin. In the patch 10 according to the second embodiment of the present disclosure, either one of the retractable patch 100 or the warming patch 200 may be in contact with the skin, and the hole H may be formed in one of the retractable patch 100 and the warming patch 200 attached to the skin. Alternatively, the hole H may be formed to penetrate both the retractable patch 100 and the warming patch 200 toward a thickness direction of the patch 10 at the same location.

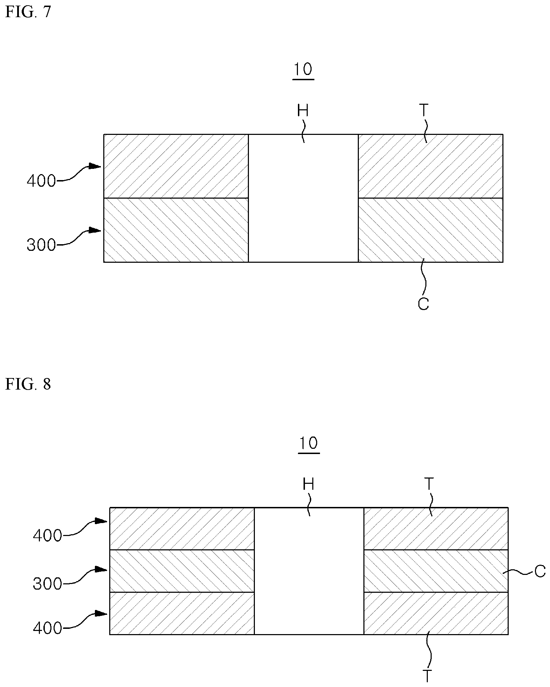

FIG. 7 is a side cross-sectional view of a part of a patch for skin stimulation according to a third embodiment of the present disclosure, and FIG. 8 is a side cross-sectional view of a part of a patch for skin stimulation according to a modified example of the third embodiment of the present disclosure.

Hereinafter, a patch for skin stimulation 10 according to the third embodiment of the present disclosure will be described in detail with reference to FIGS. 7 and 8. The patch 10 according to the third embodiment of the present disclosure is different from the first embodiment of the present disclosure in that a first layer 300 and a second layer 400 are provided. Hereinafter, for convenience of description, duplicated descriptions with the first embodiment of the present disclosure will be omitted.

The patch for skin stimulation 10 according to the third embodiment of the present disclosure may include the first layer 300 and the second layer 400. The first layer 300 and the second layer 400 are provided in combination with each other.

The first layer 300 may include a retractable material C. The retractable material C may be a material capable of retracting under a preset condition, similar to the retractable material C of the patch 10 according to the first embodiment of the present disclosure, and may be, as an example, Poly(N-isopropylacrylamide) (PNIPAAm).

The second layer 400 may include a trigger material T. Similar to the trigger material T of the patch 10 according to the first embodiment of the present disclosure, the trigger material T may be a material that enables the conditions to be achieved, may apply a specific energy to the retractable material C, and may be a water-sensitive self-heating material. The trigger material T may be a self-heating material and include zeolite. In addition, the trigger material T may include polyol.

The second layer 400 may be provided on one side of the first layer 300. The second layer 400 may be provided to be attached so as to be in surface contact with the one side of the first layer 300. The patch 10 may be in contact with a user's skin, and either the first layer 300 or the second layer 400 of the patch 10 may be in contact with the skin.

The second layer 400 may be provided on both sides of the first layer 300. For example, the second layer 400 may be provided to be attached so as to be in surface contact with both sides of the first layer 300. In this case, the second layer 400 of the patch 10 may be in contact with the user's skin.

The patch 10 according to the third embodiment of the present disclosure may include one or more holes H, similar to the patch 10 according to the first embodiment of the present disclosure. In this case, the hole H is formed in a region that is in contact with the skin. When the second layer 400 is provided on only one side of the first layer 300, the hole H may be formed on only one side of the first layer 300 or the second layer 400 or may be formed to penetrate both the first layer 300 and the second layer 400 toward the thickness direction at the same location. When the second layer 400 is provided on both sides of the first layer 300, the hole H may be formed only in the second layer 400, or the hole H may be formed to penetrate both the first layer 300 and the second layer 400 toward the thickness direction at the same location.

Hereinafter, embodiments of the present disclosure will be listed.

Item 1 is a patch for skin stimulation, as a patch capable of stimulating the skin, including: a retractable material under a preset condition; and a trigger material which applies a preset energy to the retractable material so as to achieve the condition.

Item 2 is a patch for skin stimulation including: a retractable patch including a retractable material capable of retracting under the preset condition; and a warming patch including the trigger material which applies the preset energy to the retractable material so as to achieve the condition, and the retractable patch and the warming patch are separately provided to a user before use and are used in combination with each other in use.

Item 3 is a patch for skin stimulation including: a first layer including the retractable material capable of retracting under the preset condition; and a second layer provided on one side or both sides of the first layer and including the trigger material which applies the preset energy so as to achieve the condition.

Item 4 is the patch for skin stimulation of items 1 to 3 in which the preset energy is a thermal energy.

Item 5 is the patch for skin stimulation of items 1 to 4 in which the patch is a hydrogel patch.

Item 6 is the patch for skin stimulation of items 1 to 5 in which the retractable material is Poly(N-isopropylacrylamide) (PNIPAAm).

Item 7 is the patch for skin stimulation of items 1 to 6 in which the trigger material is a water-sensitive self-heating material.

Item 8 is the patch for skin stimulation of items 1 to 7 in which the trigger material includes zeolite.

Item 9 is the patch for skin stimulation of items 1 to 8 in which the trigger material further includes polyol.

Item 10 is the patch for skin stimulation of items 1 to 9 in which the patch includes one or more holes formed in a region that is in contact with the skin.

Item 11 is the patch for skin stimulation of items 1 to 10 in which the hole is formed in a circular shape having a diameter of 1 to 5 times an average skin pore size, a plurality of holes are provided, and an interval between any two holes adjacent to each other among the plurality of holes is formed to be 3 to 5 times the diameter of the hole.

Although the embodiments of the present disclosure have been described, these are merely examples, and the present disclosure is not limited thereto, and should be interpreted to have the broadest scope in accordance with the basic idea disclosed in this specification. Those skilled in the art may combine or substitute the disclosed embodiments to implement embodiments not disclosed herein, but this does not depart from the scope of the present disclosure. In addition, those skilled in the art may easily change or modify the disclosed embodiments on the basis of this specification, and it is clear that such changes or modifications also fall within the scope of the present disclosure.

### [Reference Signs List]

10: patch
100: retractable patch
200: warming patch
300: first layer
400: second layer
C: retractable material
H: hole
T: trigger material

## Claims

1. A patch (10) for skin stimulation, as a patch capable of stimulating skin, comprising:
a retractable material capable of retracting under a preset condition; and
a trigger material which applies a preset energy to the retractable material so as to achieve the condition.

2. A patch for skin stimulation comprising:
a retractable patch including a retractable material capable of retracting under a preset condition; and
a warming patch including a trigger material which supplies a thermal energy to the retractable patch so as to achieve the condition,
wherein the retractable patch and the warming patch are separately provided to a user before use and are used in combination with each other in use.

3. A patch for skin stimulation comprising:
a first layer including a retractable material capable of retracting under a preset condition; and
a second layer provided on one side or both sides of the first layer and including a trigger material which applies a preset energy so as to achieve the condition.

4. The patch for skin stimulation of any one of claims 1 to 3, wherein the preset energy is a thermal energy.

5. The patch for skin stimulation of any one of claims 1 to 3, wherein the patch is a hydrogel patch.

6. The patch for skin stimulation of any one of claims 1 to 3, wherein the retractable material is Poly(N-isopropylacrylamide) (PNIPAAm).

7. The patch for skin stimulation of any one of claims 1 to 3, wherein the trigger material is a water-sensitive self-heating material.

8. The patch for skin stimulation of claim 7, wherein the trigger material includes zeolite.

9. The patch for skin stimulation of claim 8, wherein the trigger material further includes polyol.

10. The patch for skin stimulation of any one of claims 1 to 3, comprising one or more holes formed in a region that is in contact with the skin.
